(19) Europäisches Patentamt / European Patent Office / Office européen des brevets

(11) **EP 3 760 173 A1**

(12) **EUROPEAN PATENT APPLICATION**
published in accordance with Art. 153(4) EPC

(43) Date of publication:
**06.01.2021 Bulletin 2021/01**

(21) Application number: **19760702.1**

(22) Date of filing: **27.02.2019**

(51) Int Cl.:
*A61F 13/02* (2006.01)   *D04H 3/009* (2012.01)
*D04H 3/16* (2006.01)

(86) International application number:
**PCT/JP2019/007529**

(87) International publication number:
**WO 2019/168019 (06.09.2019 Gazette 2019/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.03.2018 JP 2018036688**

(71) Applicants:
• **Nichiban Co., Ltd.**
 **Tokyo 112-8663 (JP)**
• **KB Seiren, Ltd.**
 **Sabae-shi**
 **Fukui 916-0038 (JP)**

(72) Inventors:
• **FUJISAWA Hiromichi**
 **Tokyo 112-8663 (JP)**
• **IKAI Tomonori**
 **Tokyo 112-8663 (JP)**
• **FUKANO Kenji**
 **Tokyo 112-8663 (JP)**
• **YOSHIDA Tatsuya**
 **Tokyo 112-8663 (JP)**
• **MANABE Hiroyuki**
 **Sabae-shi, Fukui 916-0038 (JP)**

(74) Representative: **Mewburn Ellis LLP**
 **Aurora Building**
 **Counterslip**
 **Bristol BS1 6BX (GB)**

(54) **ADHESIVE BANDAGE AND POLYURETHANE NONWOVEN FABRIC FOR ADHESIVE BANDAGE**

(57) The present invention addresses the subject of providing an adhesive bandage such as a band-aid that has, as a support, a superfine urethane nonwoven fabric that has excellent air permeability and elasticity, the adhesive bandage having a good balance of various desired properties including adhesiveness to own rear surface, drying property, gas transmission, and interlaminar strength by appropriately embossing the surface of the support. The present invention is an elongated adhesive bandage comprising the superfine urethane nonwoven fabric that is embossed on one side and has an adhesive layer laminated on the side opposite the embossed side, the adhesive bandage being characterized by having a ratio (y/x) of the 30% tensile load of the long side direction (y) of the adhesive bandage to the 30% tensile load of the short side direction (x) thereof of 0.8 to 2.0, a water retention rate per weight of the urethane nonwoven fabric of 0.8 or less, an adhesive force to Bakelite plates of 5.0 to 8.0 N/24 mm, and an adhesive force to the embossed surface (adhesive force to own rear surface) of 1.2 N/24 mm or more.

EP 3 760 173 A1

**Description**

TECHNICAL FIELD

The present invention relates to an adhesive plaster, which is a medical adhesive product, for use in the protection of affected sites and wounded sites, the fixation of gauze, absorbent cotton, catheters, tubes, poultices, and the like to the skin, or the like, and a polyurethane non-woven fabric for an adhesive plaster. In particular, the present invention relates to a first-aid adhesive plaster employing a pad for protecting wounded sites, for use in the protection of sites having wounds such as cuts, chafing, stab wounds, scratched wounds, and blisters on foot, in the promotion of healing of such sites, in the relief of pains, and the like.

BACKGROUND ART

In general, an adhesive plaster has a configuration in which an adhesive agent layer is provided on one face of a support made of paper, cloth, a plastic film, or the like. For example, a flexible vinyl chloride film is excellent in toughness, stress relaxing ability, printability, and the like, and is also inexpensive, and is therefore widely used as a support in first-aid adhesive plasters. However, such a plastic film has unsatisfactory fitting ability to a skin surface, and in addition, has poor flexibility and air permeability.

By contrast, since non-woven fabrics are excellent in air permeability and do not tend to cause stuffiness upon attachment, non-woven fabrics are suitable as a support in an adhesive plaster, which is a medical adhesive product. Furthermore, in recent years, stretchable non-woven fabrics made of polyurethane or polyester have been developed, and by means of using these non-woven fabrics having both of stretching ability and air permeability as a support in an adhesive plaster, it is possible to obtain an adhesive plaster which does not tend to cause skin disorders such as rashes, itching, and erythema, and which can follow the motion of skin to which the adhesive plaster has been attached (for example, see Patent Documents 1, 2). In particular, an adhesive plaster using a polyurethane non-woven fabric and having excellent air permeability and stretching ability as a support has excellent properties (for example, see Patent Documents 3, 4). Up to now, the present applicants have proposed a first-aid adhesive plaster having a substantially rectangular shape and comprising an adhesive tape obtained by applying an adhesive agent on one face of a substrate made of a non-woven fabric having air permeability and stretching ability, wherein (1) the non-woven fabric is a polyurethane non-woven fabric which is formed of extremely ultrafine polyurethane filaments having an average fiber diameter of 7 to 15 $\mu$m, and in which a ratio (y/x) of a 50% tensile load (y) in a cross-machine direction (CD) to a 50% tensile load (x) in a machine direction (MD) is in a range of 1.10 to 2.00; and (2) the first-aid adhesive plaster is obtained by die cutting in such a way that longer sides of the substantially rectangular shape are coincident with the CD of the substrate, and shorter sides of the substantially rectangular shape are coincident with the MD of the substrate. This first-aid adhesive plaster is excellent in that such a first-aid adhesive plaster has good fitting ability and reduced discomfort feelings, and in particular, is advantageous in a viewpoint of cost because such a first-aid adhesive plaster can have reduced grammage of the non-woven fabric; and also in that, when such a first-aid adhesive plaster is wrapped around a finger or the like, the first-aid adhesive plaster is wrapped in a moderate manner, rather than in a tight or loose manner, and in addition, by contrast to conventional first-aid adhesive plasters, the wrapped first-aid adhesive plaster is not shrunk in a shorter side direction and not stretched in a longer side direction because of the relaxation of its orientation caused by increase in temperatures, or by repeating the expansion and contraction of the wrapped first-aid adhesive plaster, and as a result of this, the wrapped first-aid adhesive plaster tends not to become loose and unintentionally detached, and in addition, when the first-aid adhesive plaster is attached, peeling of the attached first-aid adhesive plaster from its edge is suppressed, and the attached first-aid adhesive plaster has good air permeability and does not inhibit cutaneous respiration, and as a result of this, it is possible to reduce uncomfortable feelings caused by stuffiness or the like, and also reduce the growth of unwanted bacteria or the like (see Patent Document 3).

Techniques of embossing a non-woven fabric have been conventionally employed, and Patent Documents 3 and 4 describe an adhesive plaster using a non-woven fabric as a support, and also describe embossing a medical adhesive tape. However, satisfactory studies have never been made about effects of conditions of the embossing process on the properties of the adhesive plaster, and an adhesive plaster having highly balanced properties have not been accomplished yet.

PRIOR ART DOCUMENTS

Patent Document

Patent Document 1: JP 2-14059 A
Patent Document 2: JP 9-560 A
Patent Document 3: JP 10-33585 A
Patent Document 4: JP 11-9623 A

SUMMARY OF INVENTION

Technical Problem

It is an object of the present invention to provide an adhesive plaster, such as a first-aid adhesive plaster, using an ultrafine urethane non-woven fabric excellent in air permeability and stretching ability as a support, wherein a surface of the support is appropriately embossed to provide the adhesive plaster having highly balanced requisite properties such as adhesion to its own back face, drying properties, air permeability, and interlaminar strength.

Solution to Problem

The present inventors have studied further improvement of the invention according to Patent Document 3, and in particular, have studied conditions of embossing. As a result, it has been found that, depending on a degree of embossing targeted for a surface of the ultrafine urethane non-woven fabric shaped into a form of a film under specific conditions, some of properties required for an adhesive plaster using the non-woven fabric as a support tend to improve, and others of these properties tend to decrease. From intense analyses and studies about these results, it has been found that by means of controlling conditions of embossing to be in a constant range, an excellent adhesive plaster is obtained, in which all of the above-described properties are in a range without any problem in a viewpoint of practical use, and the present invention has been accomplished. In other words, subjects of the present invention are as follows.

[1] An elongated adhesive plaster comprising an ultrafine urethane non-woven fabric having one surface embossed, and an adhesive agent layer stacked on a surface opposite to the embossed surface thereof, wherein a ratio (y/x) of a 30% tensile load (y) in a longer side direction to a 30% tensile load (x) in a shorter side direction of the adhesive plaster is 0.8 to 2.0, a water retention proportion of the ultrafine urethane non-woven fabric on the basis of weight is 0.8 or less, an adhesion strength of the adhesive plaster to a Bakelite plate is 5.0 to 8.0 N/24 mm, and an adhesion strength of the adhesive plaster to the embossed surface (strength of adhesion to its own back face) is 1.2 N/24 mm or more.
[2] The adhesive plaster according to the item [1], wherein an air permeation rate of the adhesive plaster is 10.0 sec/100 mL or less.
[3] The adhesive plaster according to the item [1] or [2], wherein the 30% tensile load (x) in the shorter side direction of the adhesive plaster is 2.0 to 4.0 N/24 mm, and the 30% tensile load (y) in the longer side direction of the adhesive plaster is 2.0 to 4.5 N/24 mm.
[4] A polyurethane non-woven fabric for an adhesive plaster, wherein at least one of surfaces of the polyurethane non-woven fabric is embossed, a water retention proportion on the basis of weight is 0.8 or less, a value of air permeation resistance is 1.6 kPa·s/m or less, and a ratio (Y/X) of a 30% tensile load (Y) in a cross-machine direction (CD) to a 30% tensile load (X) in a machine direction (MD) is 1.0 to 2.0.
[5] The polyurethane non-woven fabric for an adhesive plaster according to the item [4], wherein the above-described X is 2.5 to 4.0 N/24 mm, and the above-described Y is 2.5 to 4.5 N/24 mm.
[6] The polyurethane non-woven fabric for an adhesive plaster according to the item [4] or [5], wherein the polyurethane non-woven fabric has a basis weight of 50 to 100 g/m$^2$.

Advantageous Effects of Invention

According to the present invention, as a result of embossing an ultrafine urethane non-woven fabric shaped into a form of a film under specific conditions under specific conditions, it is possible to obtain an excellent adhesive plaster using the non-woven fabric as a support, in which, when such an adhesive plaster is wrapped around a finger or the like, the adhesive plaster is wrapped in a moderate manner, rather than in a tight or loose manner, and the wrapped adhesive plaster tends not to become loose and unintentionally detached because of repeating of the expansion and contraction of the wrapped adhesive plaster, and, when the adhesive plaster is attached, peeling of the attached adhesive plaster from its edge is suppressed, and in addition, strength of adhesion to its own back face, an amount of water retention (drying properties), an air permeation rate, interlaminar strength, and the like of the non-woven fabric are in a suitable range in a viewpoint of practical use. In other words, the adhesive plaster according to the present invention has excellent softness and stretching ability resulted from the ultrafine urethane non-woven fabric, and in addition,

1. Ends of the adhesive plaster are not easily peeled off, even in a case where the adhesive plaster is attached in such a way that the adhesive plaster is wrapped around a finger or the like with one end of the adhesive plaster being adhered to its own back face, and then, manual tasks and the like are performed. The interlaminar strength of the support is high, and a problem tends not to occur, in that, when the end is peeled from its own back face, delamination of the support occurs to leave an adhesive agent layer on the skin.

2. The draining properties (dry impression) immediately after doing tasks using water or taking a bath is good, and the skin with the attached adhesive plaster does not become wrinkly.

3. The air permeability of the support tends to decrease as a result of the embossing; however, in the resulting adhesive plaster, the air permeability can be in a range without any particular problem in a viewpoint of practical use, and properties required for an adhesive plaster are highly balanced.

BRIEF DESCRIPTION OF DRAWINGS

[Fig. 1] Fig. 1 shows a water retention proportion on the basis of weight at a level of each embossing depth of a urethane non-woven fabric.
[Fig. 2] Fig. 2 shows the results of evaluation in practical use regarding ease of drying of the adhesive plaster after taking a bath, at a level of each embossing depth of the urethane non-woven fabric.
[Fig. 3] Fig. 3 shows strength of adhesion to its own back face at a level of each embossing depth of a urethane non-woven fabric.
[Fig. 4] Fig. 4 shows the results of evaluation in practical use regarding adhesion ability of overlapping portions of the adhesive plaster immediately before peeling the adhesive plaster, at a level of each embossing depth of the urethane non-woven fabric.

DESCRIPTION OF EMBODIMENTS

The adhesive plaster according to the present invention, and the polyurethane non-woven fabric for the adhesive plaster according to the present invention will be described below in detail.

<Adhesive plaster>

One embodiment of the adhesive plaster of the present invention is an elongated adhesive plaster (adhesive tape), comprising a support layer (a1) having one embossed face, and including a non-woven fabric formed of ultrafine urethane filaments, and an adhesive agent layer (a2) having air permeability and stacked on a surface opposite to the embossed surface. Another embodiment of the adhesive plaster of the present invention is a so-called first-aid adhesive plaster, in which the first-aid adhesive plaster further comprises a pad layer (b) substantially located at the center of the adhesive agent layer in a slice of the adhesive plaster having a constant length. This first-aid adhesive plaster can further comprise a release sheet (c). The support layer (a1), the adhesive agent layer (a2), the pad layer (b), and the release sheet (c) constituting the adhesive plaster of the present invention will be described below in detail.

1. Support layer (a1)

The support layer includes a support layer that is obtained by embossing a non-woven fabric obtained by shaping ultrafine urethane filaments into a form of a film under specific conditions under specific conditions. In the present specification, "a non-woven fabric obtained by shaping ultrafine urethane filaments into a form of a film under specific conditions," and "a non-woven fabric formed of ultrafine urethane filaments" are also merely referred to as "an ultrafine urethane non-woven fabric." The fiber diameter of the ultrafine urethane filaments for use in shaping the ultrafine urethane non-woven fabric in the present invention into a form of a film is preferably in a range of 7 to 25 $\mu$m, and more preferably in a range of 10 to 18 $\mu$m. As a result of using such an ultrafine urethane non-woven fabric as a support layer, it is possible to obtain an adhesive plaster which has good strength, stretching ability, and appearance, which causes discomfort feelings to a reduced extent when the adhesive plaster is attached, and which highly satisfies other requisite qualities such as air permeability.

The ultrafine urethane non-woven fabric in the present invention can be suitably obtained by a method in which a thermoplastic polyurethane elastic body, which can be subjected to be melt spinning, is used to produce a non-woven fabric by a melt blowing process. The above-described thermoplastic polyurethane elastic body is obtained by melt polymerization using, as a source material, a polyol (for example, polyhexamethylene diol), a diisocyanate (for example, 4,4'-diphenylmethane diisocyanate), and a low molecular diol (for example, 1,4-butanediol) to be a hard segment.

Examples of the soft segment of polyol type in the source material of the above-described thermoplastic polyurethane elastic body include polyols with a low melting point having a number average molecular weight of 500 to 6,000, such as dihydroxypolyether, dihydroxypolyester, dihydroxycarbonate, and dihydroxypolyester amide. As the soft segment of polyether type, it is typical to use polytetramethylene glycol. Examples of the diisocyanate include 4,4'-diphenylmethane diisocyanate, tolylene diisocyanate, isophorone diisocyanate, hydrogenated diphenylmethane diisocyanate, xylylene diisocyanate, 2,6-diisocyanate methyl caproate, and hexamethylene diisocyanate. As the chain extender (hard segment), low molecular diols, amino alcohols, triols, and the like are used.

In the melt blowing process, a synthetic resin such as a thermoplastic elastomer is melted, and the resulting molten solution is dispensed from a die in the form of a nozzle having a large number of pores, and the dispensed molten solution is blown away by using a heating gas at a high speed, and collected by a net conveyor to produce an ultrafine urethane non-woven fabric. A net conveyor of endless type conveys an ultrafine urethane non-woven fabric in a direction of flow (MD). Webs collected on the net conveyor are pressed by a roller, as necessary, and are rolled up to become an ultrafine urethane non-woven fabric. In general, the ultrafine urethane non-woven fabric obtained by such a production method is oriented in a direction of product flow (MD) to not a little extent.

In the present invention, for the purpose of providing orientation in a direction perpendicular to a direction of product flow (MD) (cross-machine direction: CD), it is preferable that, in the production of the ultrafine urethane non-woven fabric by a melt blowing process, a method is employed, in which a die is moved in a back-and-forth manner toward a cross-machine direction (CD), and in addition, speed of the movement in a direction of flow (MD) in the net conveyor is adjusted to a lower speed than the speed of the back-and-forth movement of the die in a cross-machine direction. The speed of movement in a direction of flow (MD) in the net conveyor is preferably 1/3 or less, more preferably 1/25 to 1/3, and particularly preferably 1/20 to 1/5 based on the speed of back-and-forth movement of a die in a cross-machine direction (CD). When such a production method is employed, it is possible to obtain an ultrafine urethane non-woven fabric, in which, even when the non-woven fabric is formed of ultrafine filaments, the non-woven fabric has required strength, has a reduced amount of unevenness in thickness, and is oriented in a cross-machine direction. As a result of being produced in a manner described above, an ultrafine urethane non-woven fabric can be provided with ability to be cut by hand.

As an embossing process targeted for an ultrafine urethane non-woven fabric, known apparatus and method can be employed. As the embossing process, by way of example, it is possible to employ a point thermal compression bonding process. When 10 or more thermally fused points per cm$^2$ are formed by embossing, the internal strength of the ultrafine urethane non-woven fabric can be compensated. In the present invention, a large number of fine depressions and projections are formed on at least one face of the ultrafine urethane non-woven fabric to be used as a support by embossing. For the purpose of forming such fine depressions and projections, by way of example, it is preferable to employ a method in which an embossing roll having a design of depressions and projections formed on a surface, and an anvil roll are opposed to each other, and an ultrafine urethane non-woven fabric is passed between these rolls to be pressed under heating.

With regard to conditions of embossing the ultrafine urethane non-woven fabric, it is preferable that the embossing roller temperature is 80 to 150°C, the pressure (linear pressure) is 20 to 200 N/mm, and the processing rate is 5 to 20 min.

An embossed design (embossed pattern) to be provided on the ultrafine urethane non-woven fabric is not limited in particular as long as the embossed design has a shape which produces a large number of fine depressions and projections on at least one face of the ultrafine urethane non-woven fabric, and examples of such an embossed design include a lattice-like pattern, a pattern obtained by disposing a large number of short lines in a staggered manner, a pattern obtained by combining a large number of short lines with each other in a crisscross manner, and a pattern provided with a large number of small circles. Among these embossed patterns, preference is given to a lattice-like pattern having a large number of depressions defined by crossed ridge lines (strip-shaped projections). It is preferable that the line width of the ridge line is 50 to 600 μm, the distance between ridge lines (the width of the depressions) is 0.5 to 1.5 mm, and the depth of depressions is 50 to 600 μm. The total area of projections (ridge line, and the like) protruded from a back face of the ultrafine urethane non-woven fabric to be used as a support is typically 50% or less, preferably 40% or less, more preferably 30% or less, further preferably 20% or less, and particularly preferably 10% or less based on the total area of the ultrafine urethane non-woven fabric. The total area of the depressions is preferably about 5% or more, more preferably about 10% or more, and further preferably about 20% or more.

The basis weight of the ultrafine urethane non-woven fabric is typically 40 to 120 g/m$^2$, preferably 50 to 100 g/m$^2$, and more preferably in a range of 60 to 100 g/m$^2$. The thickness of the ultrafine urethane non-woven fabric is typically about 100 to 500 μm. The ultrafine urethane non-woven fabric has a water retention proportion of 0.8 or less, and preferably 0.75 or less on the basis of weight. Here, the water retention proportion on the basis of weight is determined by immersing an ultrafine urethane non-woven fabric cut into a constant size in water for 30 minutes, and subsequently, picking up the resulting ultrafine urethane non-woven fabric with a pair of tweezers, and subjecting the ultrafine urethane non-woven fabric to draining by swinging the ultrafine urethane non-woven fabric twice or three times (a situation where no water droplet runs from the ultrafine urethane non-woven fabric). Then, the water retention proportion on the basis of weight is determined from the following expression.

$$\text{(Weight after draining - Weight before immersion in water)/Weight before immersion in water}$$

When the water retention proportion of the ultrafine urethane non-woven fabric on the basis of weight is greater than 0.8, the drying properties of the adhesive plaster after taking a bath or doing tasks using water is inferior, and a skin to which the adhesive plaster is attached becomes wrinkly, and this leads to uncomfortable feelings. The lower limit of the water retention proportion of the ultrafine urethane non-woven fabric on the basis of weight is not limited in particular, and when the amount of water retention is small, the drying properties is good, and this is more preferable.

In the present invention, a value of air permeation resistance of the support (an ultrafine urethane non-woven fabric after embossing) is 1.6 kPa·s/m or less, and preferably 1.3 kPa·s/m or less. In addition, it is preferable that the value of air permeation resistance of the support is in a range of 0.1 to 1.6 kPa·s/m when the basis weight is 40 to 120 $g/m^2$, is in a range of 0.25 to 1.3 kPa·s/m when the basis weight is 50 to 100 $g/m^2$, and is in a range of 0.4 to 1.3 kPa·s/m when the basis weight is 60 to 100 $g/m^2$. It is preferable that a value obtained by dividing the value of air permeation resistance by basis weight (a value of air permeation resistance/basis weight) is in a range of 0.0040 to 0.0160 when the basis weight is 40 $g/m^2$ or more and less than 60 $g/m^2$, and in a range of 0.0075 to 0.0170 when the basis weight is 60 $g/m^2$ or more and 100 $g/m^2$ or less. When a value of air permeation resistance of the support is lower than the above-described range, the interlaminar strength of the support is unsatisfactory, and delamination tends to occur. When the value of air permeation resistance of the support is greater than the above-described range, the resulting adhesive plaster tends to cause uncomfortable residence of moisture under conditions of poor air permeation when the adhesive plaster is attached on human skin.

During the ultrafine urethane non-woven fabric is shaped into a form of a film, the support in the present invention has orientation in a cross-machine direction (CD), and as a result of this, the ratio (Y/X) of the 30% tensile load (Y) in the CD to the 30% tensile load (X) in the MD is 0.5 to 3.0, and preferably in a range of 1.0 to 2.0. X is 1.0 to 5.0 N/24 mm, and is preferably in a range of 2.5 to 4.0 N/24 mm, and Y is 1.0 to 5.0 N/24 mm, and is preferably in a range of 2.5 to 4.5 N/24 mm.

Pigments and dyes for coloring the ultrafine urethane non-woven fabric to have flesh color or the like, antistatic agents, lubricants, and the like can be added to the molten solution of polyurethane to perform a process of shaping into a form of a film, as long as effects of the present invention are not impaired.

2. Adhesive agent layer (a2)

The adhesive plaster of the present invention has a structure in which an adhesive agent layer is stacked on a surface of the support layer (a1) opposite to the embossed surface. As the adhesive agent in this adhesive agent layer (a2), an acrylic adhesive agent, an adhesive agent of rubber type, an adhesive agent of silicone type, and the like are used, and it is preferable that these agents have skin irritation to a small extent.

Examples of the acrylic adhesive agent include a homopolymer or a copolymer obtained from long-chain (meth)acrylic acid ester monomers having about 4 to 12 carbon atoms such as butyl acrylate, 2-ethylhexyl acrylate, and isononyl acrylate; or a copolymer containing monomers of (meth)acrylic acid ester type as a main component and obtained by copolymerizing one or more of other monomers to be able to be copolymerized, such as (meth)acrylic acid, vinyl acetate, styrene, vinylpyrrolidone, acrylamide, hydroxyethyl acrylate, and hydroxypropyl acrylate in a range of 2 to 50% by weight. The acrylic adhesive agent can be an acrylic adhesive agent of solvent type obtained by polymerizing the monomers using a peroxide such as benzoyl peroxide as an initiator in an organic solvent such as toluene, hexane, or ethyl acetate, under nitrogen atmosphere, or can also be an acrylic adhesive agent of emulsion type obtained by emulsifying and dispersing the monomers in water by using an emulsifier, followed by polymerization. After polymerization, it is preferable that, before application on a sheet-shaped or film-shaped substrate, an appropriate amount of a multifunctional resin such as an epoxy resin is added to crosslink the polymer.

Examples of the adhesive agent of rubber type include a composition obtained by formulating a tackifier resin, a softener, and the like into a rubber as a base material, such as synthetic polyisoprene rubber, and a styrene-isoprene-styrene block copolymer.

The adhesive agent layer (a2) in the adhesive plaster of the present invention is preferably an adhesive agent layer (a2) having air permeability. The adhesive agent layer (a2) having air permeability is an adhesive agent having an air permeation rate of 20 seconds/100 mL or less determined by using a Gurley densometer (Tester Sangyo Co., Ltd.) according to JIS-P8117. The air permeation rate of the adhesive agent layer (a2) is preferably 10 seconds/100 mL or less, more preferably 5 seconds/100 mL or less, and further preferably 1 second/100 mL or less. The air permeation rate of the adhesive agent layer (a2) itself cannot be determined, and therefore, a value of air permeation rate obtained by performing the measurement in a situation where the adhesive agent layer (a2) is laminated with a material having air permeability equivalent to or greater than that of the adhesive agent layer (a2), such as a non-woven fabric or a woven fabric, is considered as the air permeation rate of the adhesive agent layer (a2). Specifically, the measurement of the air permeation rate of the adhesive agent layer (a2) is as follows. In other words, an adhesive plaster (A) cut into a size of 50 mm × 50 mm [composed of the support layer (a1) and the adhesive agent layer (a2)] is attached to a tightening plate (base). The air-permeating area of the adhesive plaster (A) between the tightening plates is adjusted to be 645.16 mm$^2$. An inner cylinder of a Gurley densometer is pulled out, the inner cylinder is placed at a stopper, and subsequently, the specimen is tightened between the tightening plates. The inner cylinder is quietly lowered to measure the number of seconds required for the passage of air in an amount of 100 mL to obtain the air permeation rate of the adhesive plaster (a), and this air permeation rate is considered as the air permeation rate (seconds/100 mL) of the adhesive agent layer (a2). Therefore, a particularly preferable air permeation rate of the adhesive plaster (A) is 0.2 to 10.0 seconds/100 mL.

As a result of using an adhesive agent layer (a2) having air permeability as the adhesive agent layer in the adhesive plaster of the present invention, the adhesive agent layer and the adhesive plaster have good air permeability and cushioning properties, and in addition, the follow-up ability of the adhesive agent layer and the adhesive plaster to skin is improved, and adhesion properties to skin is good.

The expansion ratio of the adhesive agent layer (a2) having air permeability is typically in a range of 1.1 to 10 times, preferably in a range of 1.2 to 8 times, more preferably in a range of 1.5 to 6 times, and further preferably in a range of 2 to 5 times. The density of the adhesive agent layer (a2) having air permeability is typically in a range of 0.1 to 0.9 g/cm$^3$, preferably in a range of 0.12 to 0.8 g/cm$^3$, more preferably in a range of 0.15 to 0.6 g/cm$^3$, and further preferably in a range of 0.2 to 0.5 g/cm$^3$.

The adhesive agent layer (a2) having air permeability is typically disposed on the entire surface of one face of the support layer (a1); however, if necessary, it is also possible, instead of disposing the adhesive agent layer (a2) having air permeability on the entire surface of the support layer (a1), to dispose the adhesive agent layer (a2) having air permeability on a portion of the support layer (a1) as long as such disposition has no adverse effects on the adhesion strength.

The thickness of the adhesive agent layer (a2) having air permeability is not limited in particular, but is typically in a range of 10 to 200 μm, preferably in a range of 15 to 150 μm, and more preferably in a range of 20 to 100 μm.

The adhesion strength of the adhesive agent layer (a2) having air permeability is adjusted to be preferably in a range of 1.0 to 10 N/24 mm, and more preferably in a range of 5.0 to 8.0 N/24 mm in terms of adhesion strength to a Bakelite plate (peel force).

The adhesive agent layer (a2) having air permeability in the present invention can be obtained by an ordinary method. Specific examples of such an ordinary method include a method using foaming in which an adhesive agent is mixed with a physical blowing agent such as air, gaseous nitrogen, or gaseous carbon dioxide, or a chemical blowing agent of decomposable type, and also, an aqueous solution or an organic solvent, and subsequently, with the resulting mixture, a release paper is coated, or alternatively, a method using foaming in which with an adhesive agent, a release paper on which an aqueous solution or an organic solvent is applied or sprayed in advance is coated, followed by heating to utilize foaming or vaporization action, and also include a method in which fine holes are formed by applying moisture in the form of mist on an undried surface of the adhesive agent after the coating process, and a method in which the adhesive agent is dispensed in the form of thread and fiber onto a release paper, and as a result of these methods, an adhesive agent layer having a large number of fine holes is formed. Among these methods, the method using foaming is preferable because such a method can form fine and homogeneous holes.

3. Pad layer (b)

The adhesive plaster of the present invention can comprise a pad layer (b). In the present invention, the pad layer (b) means a layer which is disposed in order to provide functions such as hemostasis and protection targeted for an affected site, when an adhesive plaster is attached to an affected site. In general, it is possible to employ gauze, a non-woven fabric, a woven fabric, knitted fabrics, compressed fiber, hydrocolloids, and other materials having absorption ability; however, depending on sites at which the pad layer (b) is to be used, the top surface of the above-described material is, in some cases, subjected to a thin-film forming process by using polyethylene and other plastics to prevent the top surface from adhering to the affected site because of body fluid and the like resulted from the affected site. If necessary, a disinfectant, a therapeutic drug, and other drugs can be retained in the pad layer (b). The size of the pad layer (b) is typically in a range of 5% to 70%, preferably 10% to 50%, and more preferably 15% to 40% based on 100% of the area of the entirety of the adhesive plaster.

4. Release sheet (c)

Typically, the adhesive plaster of the present invention further comprises a release sheet (c). The release sheet (c) is stacked on a face of the adhesive agent layer (a2) having air permeability opposite to the support layer (a1) including the ultrafine urethane non-woven fabric. The release sheet (c) can employ one commonly used in the field of adhesive plasters, and by way of example, it is possible to employ wood-free paper subjected to a stripping process by application of a stripping agent, a paper substrate such as glassine paper, a polyester film, and the like. The release sheet (c) can be of the dimension and the shape by which the entirety of the adhesive plaster is covered with one piece of release sheet (c), or can also be greater than the area of the adhesive plaster. The release sheet (c) can be a release sheet (c) which is divided into two or more pieces, and at least one of two or more pieces of the divided release sheet (c) can be provided with a folding-back portion. Furthermore, it is also possible to employ a release sheet (c), in which the release sheet (c) is divided into three or more pieces in total as follows: one piece of release sheet (c) is disposed to cover at least a portion of the pad layer (b), and two or more pieces of release sheet (c) are disposed on the adhesive plaster and the adhesive agent layer (a2) having air permeability around the pad layer.

5. Properties of the adhesive plaster (A)

The adhesive plaster according to the present invention includes an elongated laminate including a support layer (a1) comprising an ultrafine urethane non-woven fabric having one face embossed, and an adhesive agent layer (a2) having air permeability stacked on a face opposite to the embossed surface.

It is preferable that a ratio (y/x) of the 30% tensile load (y) in the case where longer sides of the elongated adhesive plaster are set to be along a cross-machine direction (CD) of the original fabric in relation to the 30% tensile load (x) in the case where shorter sides of the elongated adhesive plaster are set to be along a machine direction (MD) of the original fabric is in a range of 0.5 to 3.0, and preferably in a range of 0.8 to 2.0. x is 1.0 to 5.0 N/24 mm, and preferably 2.0 to 4.0 N/24 mm. y is 1.0 to 5.0 N/24 mm, and preferably 2.0 to 4.5 N/24 mm. As a result of the fact that the above-mentioned numerical values are in the respective ranges mentioned above, the following effects can be provided: when the elongated adhesive plaster is wrapped around a finger or the like, the elongated adhesive plaster is wrapped in a moderate manner, rather than in a tight or loose manner, and the wrapped elongated adhesive plaster tends not to become loose and unintentionally detached because of repeating of the expansion and contraction of the wrapped elongated adhesive plaster, and when the elongated adhesive plaster is attached, peeling of the attached elongated adhesive plaster from its edge is suppressed.

The adhesion strength of the adhesive plaster (A) to a Bakelite plate is 5.0 to 8.0 N/24 mm, and preferably 6.5 to 8.0 N/24 mm. The adhesion strength of the adhesive plaster (A) to the embossed surface of the support (strength of adhesion to its own back face) is 1.2 N/24 mm or more, and preferably 1.3 N/24 mm or more. Here, the measurement of the adhesion strength of the adhesive plaster to a Bakelite plate is performed according to JIS-Z0237. The adhesion strength of the adhesive plaster to the embossed surface of the support (strength of adhesion to its own back face) is measured as follows: a slice of the adhesive plaster having a width of 24 mm and a length of 100 mm is laminated on a Bakelite plate, and then, on a back face of the Bakelite plate, an adhesive-agent carrying face of a slice of the adhesive plaster having a width of 24 mm and a length of 240 mm is laminated in such a way that the end of the adhesive-agent carrying face of the slice and the end the back face are overlapped to be aligned with each other, and after pressurization at a load of 50 g once, peel force from the back face of the slice toward a direction of 180 degrees is measured by using a tensile tester at a drawing rate of 300 mm/min.

When the adhesion strength of the adhesive plaster (A) to a Bakelite plate is less than 5.0, the adhesion strength of the attached adhesive plaster (A) to skin is decreased, and the attached adhesive plaster (A) is peeled because of friction with clothing, and in a case where the adhesive plaster (A) is attached to bendable and extendable sites such as joint sites, uplift from the skin during the attachment may occur. When the adhesion strength of the adhesive plaster (A) to a Bakelite plate is greater than 8.0, peeling in the attachment tends not to occur, but peeling of the attached adhesive plaster (A) tends to strip off keratin of the skin portion with the attached adhesive plaster (A), and as a result of this, pain is identified. In a case where the strength of adhesion to its own back face is less than 1.2, and where the adhesive plaster (A) is attached in such a way that the adhesive plaster (A) is wrapped around a finger or the like with one end of the adhesive plaster (A) being attached to its own back face, peeling tends to occur because of friction with clothing, and the like during the attachment. The upper limit of the strength of adhesion to its own back face is not limited in particular, but is preferably 2.0 N/24 mm. When the strength of adhesion to its own back face is greater than 2.0, the attached adhesive plaster (A) tends to be difficult to peel, for example, after usage, or the surface layer of the non-woven fabric tends to be peeled.

The adhesive plaster (A) preferably has an air permeation rate of 10.0 seconds/100 mL or less. When the air permeation rate is greater than 10.0 seconds/100 mL, there is a tendency in that uncomfortable residence of moisture under conditions of poor air permeation tends to occur in a case where the adhesive plaster (A) is attached in summer or the like.

The adhesive plaster (A) according to the present invention satisfying all of the above-described numerical ranges regarding the respective properties can be obtained by principally controlling the type, the size, the shape, and the like of the embossed design (embossed pattern) possessed by the support layer (a1), and heating, compression bonding force (pressure), and the like in the embossing, and in addition, controlling the adhesion strength of the adhesive layer (a2).

The adhesive plaster (A) of the present invention satisfying all of the above-described numerical ranges regarding the respective properties has excellent softness and stretching ability in the support layer (a1), which are resulted from the ultrafine urethane non-woven fabric, and in addition, has the following features.

1. Ends of the adhesive plaster are not easily peeled off, even in a case where the adhesive plaster is attached in such a way that the adhesive plaster is wrapped around a finger or the like with one end of the adhesive plaster being adhered to its own back face, and then, manual tasks are performed. The interlaminar strength of the support is high, and a problem tends not to occur, in that, when the end is peeled from its own back face, delamination of the support occurs to leave an adhesive agent layer on the skin.
2. The draining properties (dry impression) immediately after doing tasks using water or taking a bath is good, and skin with the attached adhesive plaster does not become wrinkly.
3. The air permeability of the support tends to decrease as a result of the embossing; however, in the resulting adhesive plaster, the air permeability can be in a range without any particular problem in a viewpoint of practical use, and properties required for an adhesive plaster are highly balanced.

6. Method for producing the adhesive plaster (A)

The method for producing the adhesive plaster (A) of the present invention is not limited in particular; however, it is also possible to form an foamed adhesive agent layer (a2) by embossing one face of a support layer (a1) comprising a non-woven fabric formed of ultrafine urethane fibers, and subsequently, directly coating a face opposite to the embossed surface with a solution or an emulsion of an adhesive agent component in an organic solvent to foam the adhesive agent. It is also possible to employ a method in which a process paper to which a stripping agent of silicone type, or the like is applied is coated with a solution or an emulsion of an adhesive agent in an organic solvent, and the adhesive agent is foamed to form a foamed adhesive agent layer (a2), and subsequently, the foamed adhesive agent layer (a2) is laminated with a support layer (a1) comprising a non-woven fabric formed of ultrafine urethane fibers. In a case where the adhesive agent layer (a2) is not disposed on the entire surface of the support layer (a1), or otherwise if needed, the coating with the solution or emulsion of the synthetic resin to be used as an adhesive agent can be conducted by employing a spot-like pattern, a striped pattern, or another pattern. The end-product form of the adhesive plaster (A) is the form of a roll obtained as follows: a laminate obtained by laminating a support layer (a1), an adhesive agent layer (a2), and a release sheet (c) in this order is cut with a constant width (in an arbitrary range of about 5 mm to 100 mm) in a cross-machine direction in the coating, followed by rolling up to have an appropriate length, or is the form of a slice obtained by cutting such a laminate to have an appropriate length (in an arbitrary range of approximately a few centimeters to 1 m).

In the case where the adhesive plaster of the present invention is a first-aid adhesive plaster (B), the production method is such that a pad layer (b) is cut into a suitable size, and the cut pad layer (b) is disposed substantially at the center of the adhesive-agent carrying face of a slice obtained from the adhesive plaster (A) and having any length in a range of approximately a few centimeters to 10 cm, and in a typical manner, a release paper (c) is further disposed, and then, the shape of the first-aid adhesive plaster (typically, a rectangle having a circle-shaped periphery on the four corners) is cut out in such a way that a longer side direction is aligned with a cross-machine direction in the coating, and as a result of this, an adhesive plaster is obtained. Typically, in a subsequent step, the first-aid adhesive plaster is encapsulated in wrapping paper formed of paper, a plastic film, or a composite of these to be in a form of product.

<Polyurethane non-woven fabric for an adhesive plaster>

The present invention also includes a polyurethane non-woven fabric for suitable use as a support layer in the above-described adhesive plaster of the present invention. The polyurethane non-woven fabric for an adhesive plaster of the present invention is stated as "an ultrafine urethane non-woven fabric" in the item of Adhesive plaster, and with regard to the specific contents regarding such a polyurethane non-woven fabric, the statement regarding "an ultrafine urethane non-woven fabric" in the item of Adhesive plaster can be applied.

A preferable embodiment as a polyurethane non-woven fabric for an adhesive plaster of the present invention is a polyurethane non-woven fabric, wherein at least one of surfaces of the polyurethane non-woven fabric is embossed, a water retention proportion on the basis of weight is 0.8 or less, a value of air permeation resistance is 1.6 kPa·s/m or less, and a ratio (Y/X) of a 30% tensile load (Y) in a cross-machine direction (CD) to a 30% tensile load (X) in a machine direction (MD) is 1.0 to 2.0. It is preferable that the above-described X is 2.5 to 4.0 N/24 mm, and the above-described Y is 2.5 to 4.5 N/24 mm. The basis weight is preferably 50 to 100 g/m$^2$. As a result of using such a polyurethane non-woven fabric (ultrafine urethane non-woven fabric) as a support layer for the adhesive plaster, it is possible to obtain an adhesive plaster which has good strength, stretching ability, and appearance, which causes discomfort feelings to a reduced extent when the adhesive plaster is attached, and which highly satisfies other requisite qualities such as air permeability.

EXAMPLES

The present invention will be described below in a more detailed manner with reference to Examples; however, the present invention is not limited by these Examples in any way.

<Production of adhesive plaster>

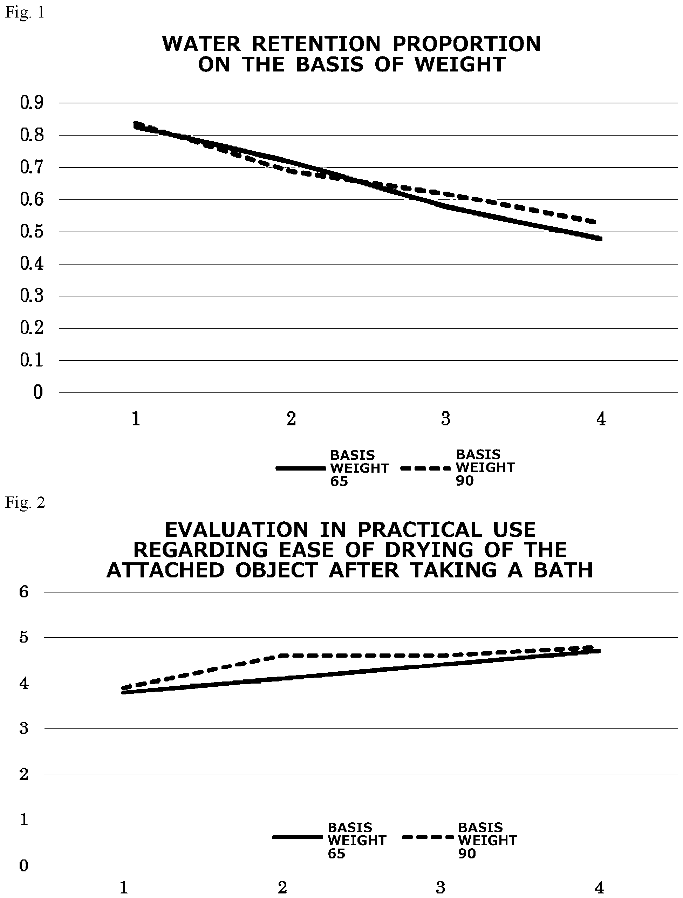

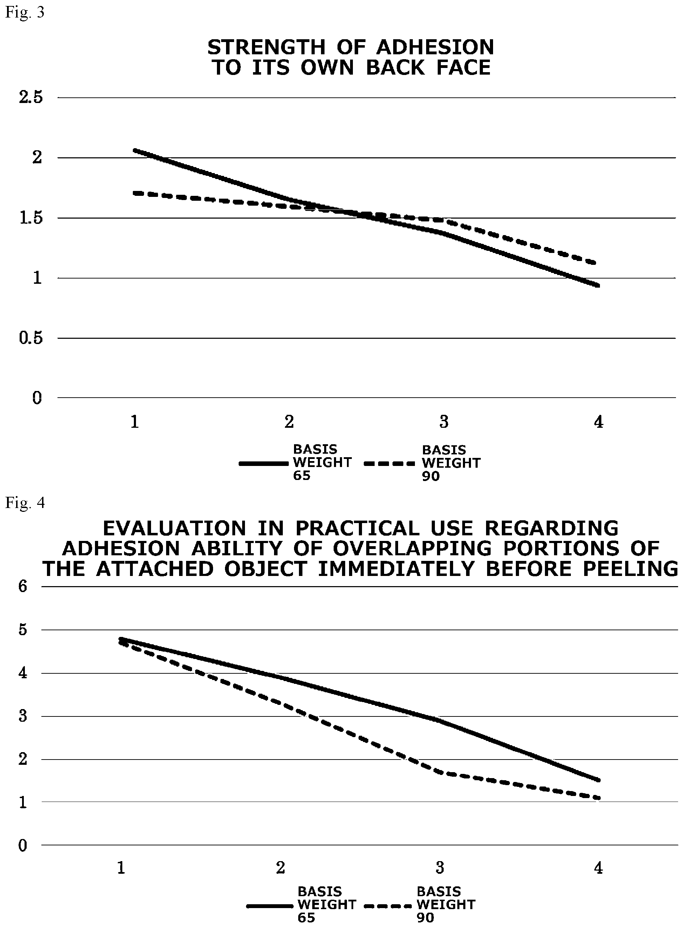

Polyhexamethylene diol having a number average molecular weight of 2,000, 4,4-diphenylmethane diisocyanate, and 1,4-butane diol were subjected to melt polymerization by using a twin-screw polymerization machine to obtain a polyether polyurethane elastic body having Shore A hardness of 90. This polyurethane elastic body was subjected to melt blow spinning, a group of the resulting molten fibers was dropped on a net conveyor, and melt-blown non-woven fabrics (a non-woven fabric made of ultrafine urethane fibers) having a basis weight of 65 g/m$^2$ (original fabric 1) and 90 g/m$^2$ (original fabric 2) were obtained. The average fiber diameter of the polyurethane fibers forming these melt-blown non-woven fabrics was 10.6 $\mu$m. The resulting non-woven fabrics made of ultrafine urethane fibers were subjected to thermal embossing by a point thermal compression bonding process to obtain a support layer (a1) of the adhesive plaster.
With regard to conditions of the embossing, the embossed design (embossed pattern) is diagonal lattice-like pattern having a large number of depressions defined by crossed ridge lines (strip-shaped projections), the line width of the ridge line is 70 $\mu$m, the distance between ridge lines (the width of the depressions) is 0.99 mm, and the depth of the depressions is 470 $\mu$m. The total area of projections (ridge line, and the like) protruded from a back face of the non-woven fabric to be used as a support is 7% based on the total area of the non-woven fabric, and the total area of the depressions is 25%. The spot to be subjected to thermal compression bonding employed a pitch of 1.98 mm in both of the MD (machine direction) and the CD (cross-machine direction).
In the thermal embossing, both of the original fabrics 1 and 2 were subjected to a constant pressure of 45 kN (linear pressure of 30 N/mm) with the temperatures of the thermal embossing varying in four stages, and the resulting original fabrics 1 and 2 were assigned according to the ascending order of such temperatures as 1-1, 1-2, 1-3, and 1-4 (these were original fabrics 1), and as 2-1, 2-2, 2-3, and 2-4 (these were original fabrics 2). The processing temperatures and the embossing depth correlated with each other, and as the processing temperature became higher, the embossing depth became deeper. With regard to the original fabric 1, the embossing depth was considered as good in the case where the value of air permeation resistance was 0.5 to 1.1, and with regard to the original fabric 2, the embossing depth was considered as good in the case where the value of air permeation resistance was 0.75 to 1.3.
With a solution of an acrylic adhesive agent [100 parts by mass of 2-ethylhexyl acrylate/vinyl acetate/acrylic acid (87/10/3) to which 0.03 parts by mass of, a cross-linking agent of epoxy type (Tetrad X manufactured by Mitsubishi Gas Chemical Company, Inc.) was added], a process paper subjected to silicone treatment was coated, to have a thickness after drying of 40 $\mu$m, and as a process for impartment of air permeability, distilled water was sprayed on the undried coated surface, followed by heating at 130°C to form fine holes, and as a result of this, an adhesive agent layer (a2) composed of an acrylic resin having air permeability was formed. Then, on the adhesive agent layer (a2), the above-described non-woven fabric made of ultrafine urethane fibers was laminated to produce an adhesive plaster (A).
A pad layer (b) composed of gauze was cut to have a width of 13 mm and a length of 22 mm in advance, and the cut pad layer (b) was placed on an acrylic adhesive agent layer (a2) having air permeability and exposed by peeling the process paper from the adhesive plaster (A), with spacing of 12 mm in the MD and 50 mm in the CD on a surface of the original fabric in such a way that the cut pad layer (b) was opposed to the adhesive agent layer (a2). Subsequently, the adhesive plaster (A) and the pad layer (b) were covered with a release paper (c) composed of glassine paper subjected to a stripping process by using a resin of silicone type, and in a situation where the pad layer (b) was located substantially at the center, die cutting was performed in such a way that a longer side direction was aligned with a cross-machine direction of the original fabric to achieve a substantially rectangular shape having a width of 25 mm and a length of 72 mm and also having rounded corners, and as a result of this, a first-aid adhesive plaster (B) was obtained. The ratio of the area of the pad layer (b) to the area of the adhesive plaster (A) was about 16%. The results obtained by determining the properties of this first-aid adhesive plaster, and the results of evaluation in practical use are shown in Table 1 and Figs. 1 to 4. When the numerical values of the 30% tensile load for the non-woven fabric and the adhesive plaster are compared with each other, there is a tendency in that the numerical value for the adhesive plaster is slightly higher than that for the non-woven fabric in the MD, and the numerical value for the non-woven fabric is slightly higher than that for the adhesive plaster in the CD. Such a tendency is attributed to the fact that tensile force in the MD was generated in the processing for impartment of adhesion ability, and as a result of this, the orientation of the ultrafine urethane filaments was changed.

<Evaluation of properties>

The properties of the non-woven fabrics and the adhesive plasters were evaluated by the following method.

(1) Atmosphere for the test

The measurement was performed at a temperature of 23 ±2°C, and a relative humidity of 50 ±5%. The sample was subjected to conditioning for 24 hours in an atmosphere of such a temperature and such a relative humidity in advance, and the specimen was subjected to the test 30 minutes after the specimen was laminated with an object as an adherent.

(2) Basis weight

The weight of the specimen of 250 mm × 250 mm was determined according to JIS-L1913 "Test methods for nonwovens," and the obtained weight of the specimen was converted to the weight per $m^2$.

(3) Value of air permeation resistance

By means of using an air permeability tester (KES-F8-AP1) manufactured by KATO TECH Co., Ltd., a stack of two pieces of samples was fixed in the apparatus, and the air permeation resistance for 6 seconds as a total of the exhaust for 3 seconds and the intake for 3 seconds was determined.

(4) Amount of water retention

A non-woven fabric, which was cut to have a constant size (100 mm × 100 mm), and the weight of which was determined, was immersed in water for 30 minutes, and subsequently, was picked up with a pair of tweezers, and the non-woven fabric was subjected to draining by swinging the non-woven fabric twice or three times (a situation where no water droplet runs from the non-woven fabric). Then, after quick weighing, the water retention proportion on the basis of weight was determined according to the following expression.

$$(\text{Weight after draining - Weight before immersion in water})/\text{Weight before immersion in water}$$

(5) 30% tensile load

According to JIS-K7115, in a situation where the MD or the CD was considered as a longer side, both ends of a slice of the original fabric of the non-woven fabric having a width of 24 mm and a length of 150 mm were held in a straight manner by a tensile tester with spacing of 100 mm, and were drawn at a drawing rate of 300 mm/min to read the stress at the time point of the drawing of 30 mm.

(6) The thickness of the adhesive plaster

The sample was subjected to five-point measurement using a dial gauge (1/100, probe diameter: 5 mm in diameter) to obtain an average value of this five-point measurement.

(7) Grammage of the adhesive plaster

The grammage was determined by weighing the sample cut into a square with 100 mm by 100 mm.

(8) Adhesion strength to a Bakelite plate

With regard to the adhesion strength of the adhesive plaster, the adhesive plaster (A) cut to have a width of 24 mm × a length of 250 mm was used as a specimen. Onto a test panel made of Bakelite and subjected to surface cleaning treatment in advance, an adhesive-agent carrying face of the specimen was pressed and attached, and subsequently, lamination was performed by using a roller of 2 kg at a compression bonding rate of 300 mm/min with the number of times of the compression bonding being one time of go-and-return movement, and as a result of this, a specimen was prepared. After the passage of 20 minutes from the lamination, adhesion strength to a Bakelite plate was determined according to JIS-Z0237 under conditions of a peeling angle of 180° and a peeling rate of 300 mm/min.

(9) Adhesion strength of the adhesive agent to the embossed surface of the support (strength of adhesion to its own back face)

A slice of the adhesive plaster having a width of 24 mm (in the MD of the original fabric) and a length of 100 mm (in the CD of the original fabric) was laminated on a Bakelite place, and then, on a back face of the Bakelite plate, an adhesive-agent carrying face of a slice of the adhesive plaster having a width of 24 mm and a length of 240 mm was laminated in such a way that the end of the adhesive-agent carrying face of the slice and the end of the back face were overlapped to be aligned with each other, and after pressurization at a load of 50 g once, peel force from the back face of the slice toward a direction of 180 degrees was measured by using a tensile tester at a drawing rate of 300 mm/min.

(10) Air permeation rate of the adhesive plaster (A)

The air permeation rate of the adhesive plaster (A) was determined by using a Gurley densometer (Tester Sangyo Co., Ltd.) according to JIS-P8117. Specifically, the adhesive plaster (A) cut into a size of 50 mm × 50 mm was attached to a tightening plate (base) with the proviso that, if a release sheet was present, the release sheet was peeled before attaching the adhesive plaster (A). The air-permeating area of the adhesive plaster (A) between the tightening plates was adjusted to be 645.16 mm$^2$. An inner cylinder of a Gurley densometer was pulled out, the inner cylinder was placed at a stopper, and subsequently, the specimen was tightened between the tightening plates, and the inner cylinder was quietly lowered to measure the number of seconds required for the passage of air in an amount of 100 mL to obtain an air permeation rate (seconds/100 mL).

<Evaluation in practical use>

Volunteers of 10 persons at total including healthy five men and healthy women underwent the attachment of a sample of the first-aid adhesive plaster (B) in such a way that a sample of the first-aid adhesive plaster (B) was wrapped around the distal interphalangeal joint of the index finger with a pad layer being located on the side of the back of the hand, and in addition, an adhesive-agent carrying face of one end of the sample was overlapped on a surface of the first-aid adhesive plaster, and after the passage of 24 hours including taking a bath at the night of the day of the attachment, the sample was peeled, and during this period of time, self-evaluation regarding a variety of states described below with a scale of 4 to 6 stages was performed, and the average values from this self-evaluation were determined.

(1) Discomfort feelings (immediately after attachment, and during attachment)

    5: Without discomfort feelings
    4: Discomfort feelings exist in some degree
    3: Discomfort feelings exist in some degree, but are not problematic
    2: Discomfort feelings exist, and are problematic
    1: Discomfort feelings exist in large extent
    0: Peeled because of excessive discomfort feelings

(2) Itching during attachment

    5: No itching
    4: Itchy in some degree
    3: Itchy, but not problematic
    2: Itchy, and problematic
    1: Itchy, and very problematic

0: Peeled because of excessive itching

(3) Ease of drying of the adhesive plaster after taking a bath

5: Not annoying
4: Dried in a slightly slow manner, but not annoying
3: Cannot determine whether or not drying was achieved in a slow manner
2: Dried in a somewhat slow manner, and annoying
1: Slow, and annoying

(4) Adhesion ability to skin immediately before peeling, and adhesion ability of overlapping portions of the adhesive plaster immediately before peeling

5: Adhered well on the entirety of the surface
4: Slightly peeled at edges
3: Approximately one third was peeled
2: Approximately the half was peeled
1: Peeled enough to cause curling up of the pad
0: Peeled, and detached

(5) Peeled state of overlapping portions of the adhesive plaster after peeling (evaluation of interlaminar strength)

5: No delamination, and the surface of the adhesive plaster was peeled perfectly
4: Delamination of a portion of the surface layer of the adhesive plaster occurred
3: In the area equal to or greater than the half of the entire area, the delamination of the surface layer of the adhesive plaster occurred
2: On the entirety of the surface, the delamination of the surface layer of the adhesive plaster occurred

(6) Wrinkled state of skin immediately after peeling

5: No wrinkled state
4: Wrinkled state was present in some degree
3: Wrinkled state, but not problematic
2: Wrinkled state, and burning pains
1: Excessively wrinkled state, and skin peeled off

[Table 1]

| Item | | Unit | | Comparative Example 1 | Example 1 | Example 2 | Comparative Example 2 | Comparative Example 3 | Example 3 | Example 4 | Comparative Example 4 |
|---|---|---|---|---|---|---|---|---|---|---|---|
| | | | | Original fabric 1-1 | Original fabric 1-2 | Original fabric 1-3 | Original fabric 1-4 | Original fabric 2-1 | Original fabric 2-2 | Original fabric 2-3 | Original fabric 2-4 |
| Support (non-woven fabric after embossing) | Basis weight | g/m² | | 64.6 | 65.8 | 65.9 | 67.9 | 91.2 | 91 | 87.7 | 91.7 |
| | Value of air permeation resistance | kPa·s/m | | 0.457 | 0.594 | 1.060 | 1.318 | 0.554 | 0.794 | 1.292 | 1.583 |
| | Water retention proportion on the basis of weight | | Average | 0.83 | 0.72 | 0.58 | 0.48 | 0.84 | 0.69 | 0.62 | 0.53 |
| | | | R | 0.20 | 0.02 | 0.09 | 0.06 | 0.03 | 0.08 | 0.04 | 0.10 |
| | 30% tensile load [MD] (X) | N/24mm | Average | 2.60 | 2.70 | 2.81 | 2.83 | 3.25 | 3.42 | 3.53 | 3.67 |
| | | | R | 0.41 | 0.55 | 0.39 | 0.58 | 0.48 | 0.68 | 0.41 | 0.22 |
| | 30% tensile load [CD] (Y) | N/24mm | Average | 2.82 | 3.13 | 3.06 | 3.18 | 4.23 | 4.23 | 4.15 | 4.35 |
| | | | R | 0.09 | 0.83 | 0.20 | 0.32 | 0.24 | 0.24 | 0.13 | 0.59 |
| | (Y)/(X) | | | 1.08 | 1.16 | 1.09 | 1.12 | 1.30 | 1.24 | 1.18 | 1.19 |
| Adhesive plaster | Total thickness | mm | Average | 0.18 | 0.18 | 0.18 | 0.19 | 0.27 | 0.25 | 0.24 | 0.26 |
| | | | R | 0.03 | 0.03 | 0.04 | 0.02 | 0.03 | 0.03 | 0.02 | 0.01 |
| | Grammage | g/m² | Average | 95.6 | 98.8 | 104.4 | 100.8 | 123.2 | 126.0 | 121.6 | 127.3 |
| | | | R | 11.2 | 8.1 | 17.1 | 14.1 | 12.9 | 6.6 | 3.2 | 11.7 |
| | 30% tensile load [in a shorter side direction] (x) | N/24mm | Average | 3.15 | 2.92 | 3.42 | 3.02 | 3.78 | 3.61 | 3.57 | 3.82 |
| | | | R | 0.56 | 0.26 | 0.48 | 0.38 | 0.17 | 0.84 | 0.25 | 0.18 |
| | 30% tensile load [in a longer side direction] (y) | N/24mm | Average | 2.71 | 2.47 | 3.25 | 2.99 | 3.72 | 3.82 | 3.95 | 4.10 |
| | | | R | 0.41 | 1.21 | 0.49 | 0.33 | 0.66 | 0.25 | 0.08 | 0.73 |
| | (y)/(x) | | | 0.86 | 0.85 | 0.95 | 0.99 | 0.98 | 1.06 | 1.11 | 1.07 |
| | Adhesion strength to a Bakelite plate | N/24mm | Average | 7.66 | 7.43 | 7.74 | 5.46 | 8.21 | 7.63 | 6.72 | 6.32 |
| | | | R | 2.25 | 0.41 | 1.58 | 0.12 | 1.99 | 2.42 | 0.57 | 0.70 |
| | Strength of adhesion to its own back face | N/24mm | Average | 2.06 | 1.65 | 1.37 | 0.94 | 1.71 | 1.59 | 1.48 | 1.12 |
| | | | R | 0.17 | 0.46 | 0.43 | 0.37 | 0.23 | 0.59 | 0.08 | 0.53 |
| | Air permeation rate | sec/100mL | Average | 0.2 | 0.3 | 0.6 | 0.5 | 0.2 | 0.2 | 0.3 | 0.3 |
| | | | R | 0.0 | 0.0 | 0.5 | 0.2 | 0.0 | 0.0 | 0.1 | 0.1 |
| Discomfort feelings immediately after attachment | | | | 4.1 | 4.0 | 4.0 | 4.0 | 3.9 | 3.8 | 3.8 | 3.7 |
| Discomfort feelings during attachment | | | | 4.1 | 4.1 | 4.3 | 4.1 | 4.0 | 4.0 | 4.0 | 4.0 |
| Itching during attachment | | | | 4.9 | 5.0 | 5.0 | 4.9 | 5.0 | 5.0 | 5.0 | 5.0 |
| Ease of drying of the attached object after taking a bath | | | | 3.8 | 4.1 | 4.4 | 4.7 | 3.9 | 4.6 | 4.6 | 4.8 |
| Adhesion ability to skin immediately before peeling | | | | 4.3 | 4.0 | 4.3 | 3.7 | 3.2 | 3.8 | 2.8 | 3.1 |
| Adhesion ability of overlapping portions of the attached object immediately before peeling | | | | 4.8 | 3.9 | 2.9 | 1.5 | 4.7 | 3.3 | 1.7 | 1.1 |
| Peeled state of overlapping portions of the attached object after peeling | | | | 5.0 | 5.0 | 5.0 | 5.0 | 5.0 | 5.0 | 5.0 | 5.0 |
| Wrinkled state of skin immediately after peeling | | | | 4.9 | 4.9 | 4.8 | 4.8 | 4.9 | 4.9 | 4.9 | 4.9 |

From the results shown in Table 1 and Figs. 1 to 4 (the numerals on the horizontal axes in these figures represent the numbers suffixed to the original fabrics, and the numerals on the vertical axes represent the numerical values of each of the evaluation items, and the graphs are obtained by making a line connecting the average values obtained from the results of the evaluation of the respective original fabrics), the following is derived. That is, with regard to both of the basis weight of 65 and the basis weight of 90, there is a tendency in that, in the evaluation in practical use, (i) the ease of drying of the adhesive plaster after taking a bath becomes better as embossment on the surface of non-woven fabric becomes deeper, and this tendency has been identified to correlate with the amount of water retention on the basis of weight. In this time, (ii) there is a tendency in that adhesion ability of overlapping portions of the adhesive plaster immediately before peeling is decreased, and this tendency has been identified to correlate with the strength of adhesion to its own back face.

With reference to the results of the respective examples, in Examples 1 to 4 using the original fabrics 1-2, 1-3, 2-2, and 2-3 as the supports, the ease of drying of the adhesive plaster after taking a bath was excellent because the average value determined by the 10 persons in the practical-use test was 4.0 or more, and in addition, the adhesion ability of overlapping portions of the adhesive plaster immediately before peeling was excellent because the result of the practical-use test was 1.7 or more, and other properties, i.e., the discomfort feelings immediately after attachment, the discomfort feelings during attachment, the itching during attachment, the adhesion ability to skin immediately before peeling, the peeled state of overlapping portions of the adhesive plaster after peeling, and the wrinkled state of skin immediately after peeling were in a range without any problem, and therefore, Examples 1 to 4 were adhesive plasters having highly balanced properties in a viewpoint of practical use.

With regard to all of Comparative Examples 1 to 4 using the original fabrics 1-1, 1-4, 2-1, and 2-4 as the supports, there was no problem regarding the discomfort feelings immediately after attachment, the discomfort feelings during attachment, the itching during attachment, the adhesion ability to skin immediately before peeling, the peeled state of overlapping portions of the adhesive plaster after peeling, and the wrinkled state of skin immediately after peeling; however, with regard to Comparative Examples 1 and 3, the degree (depth) of embossing was relatively low, and although the adhesion ability of overlapping portions of the adhesive plaster immediately before peeling was excellent, the ease of drying of the adhesive plaster after taking a bath was slightly poor. With regard to Comparative Examples 2 and 4, the degree (depth) of embossing was relatively large, and the ease of drying of the adhesive plaster after taking a bath was excellent; however, the adhesion ability of overlapping portions of the adhesive plaster immediately before peeling was slightly poor, and with regard to all of Comparative Examples, the balance between properties was worse than that of the adhesive plasters according to the present invention, in a viewpoint of practical use.

INDUSTRIAL APPLICABILITY

According to the present invention, as a result of embossing an ultrafine urethane non-woven fabric shaped into a form of a film under specific conditions under specific conditions, it is possible to obtain an excellent adhesive plaster using the non-woven fabric as a support, in which, when such an adhesive plaster is wrapped around a finger or the like, the adhesive plaster is wrapped in a moderate manner, rather than in a tight or loose manner, and the wrapped adhesive plaster tends not to become loose and unintentionally detached because of repeating of the expansion and contraction of the wrapped adhesive plaster, and when the adhesive plaster is attached, peeling of the attached adhesive plaster from its edge is suppressed, and in addition, strength of adhesion to its own back face, an amount of water retention (drying properties), an air permeation rate, interlaminar strength, and the like of the non-woven fabric are in a suitable range in a viewpoint of practical use.

**Claims**

1. An elongated adhesive plaster comprising an ultrafine urethane non-woven fabric having one surface embossed, and an adhesive agent layer stacked on a surface opposite to the embossed surface thereof, wherein
   a ratio (y/x) of a 30% tensile load (y) in a longer side direction to a 30% tensile load (x) in a shorter side direction of the adhesive plaster is 0.8 to 2.0,
   a water retention proportion of the ultrafine urethane non-woven fabric on the basis of weight is 0.8 or less,
   an adhesion strength of the adhesive plaster to a Bakelite plate is 5.0 to 8.0 N/24 mm, and
   an adhesion strength of the adhesive plaster to the embossed surface (strength of adhesion to its own back face) is 1.2 N/24 mm or more.

2. The adhesive plaster according to claim 1, wherein an air permeation rate of the adhesive plaster is 10.0 sec/100 mL or less.

3. The adhesive plaster according to claim 1 or 2, wherein the 30% tensile load (x) in the shorter side direction of the adhesive plaster is 2.0 to 4.0 N/24 mm, and the 30% tensile load (y) in the longer side direction of the adhesive plaster is 2.0 to 4.5 N/24 mm.

4. A polyurethane non-woven fabric for an adhesive plaster, wherein
   at least one of surfaces of the polyurethane non-woven fabric is embossed,
   a water retention proportion on the basis of weight is 0.8 or less,
   a value of air permeation resistance is 1.6 kPa·s/m or less, and
   a ratio (Y/X) of a 30% tensile load (Y) in a cross-machine direction (CD) to a 30% tensile load (X) in a machine direction (MD) is 1.0 to 2.0.

5. The polyurethane non-woven fabric for an adhesive plaster according to claim 4, wherein
   the X is 2.5 to 4.0 N/24 mm, and
   the Y is 2.5 to 4.5 N/24 mm.

6. The polyurethane non-woven fabric for an adhesive plaster according to claim 4 or 5, wherein the polyurethane non-woven fabric has a basis weight of 50 to 100 $g/m^2$.

Fig. 1

## WATER RETENTION PROPORTION
## ON THE BASIS OF WEIGHT

BASIS WEIGHT 65    BASIS WEIGHT 90

Fig. 2

## EVALUATION IN PRACTICAL USE
## REGARDING EASE OF DRYING OF THE
## ATTACHED OBJECT AFTER TAKING A BATH

BASIS WEIGHT 65    BASIS WEIGHT 90

Fig. 3

## STRENGTH OF ADHESION
## TO ITS OWN BACK FACE

BASIS WEIGHT 65    BASIS WEIGHT 90

Fig. 4

## EVALUATION IN PRACTICAL USE REGARDING
## ADHESION ABILITY OF OVERLAPPING PORTIONS OF
## THE ATTACHED OBJECT IMMEDIATELY BEFORE PEELING

BASIS WEIGHT 65    BASIS WEIGHT 90

## INTERNATIONAL SEARCH REPORT

| International application No. |
| --- |
| PCT/JP2019/007529 |

A. CLASSIFICATION OF SUBJECT MATTER
Int.Cl. A61F13/02(2006.01)i, D04H3/009(2012.01)i, D04H3/16(2006.01)i

According to International Patent Classification (IPC) or to both national classification and IPC

B. FIELDS SEARCHED

Minimum documentation searched (classification system followed by classification symbols)
Int.Cl. A61F13/02, D04H3/009, D04H3/16

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 10-33585 A (NICHIBAN CO., LTD.) 10 February 1998, paragraphs [0011]-[0024], table 2 (Family: none) | 1-6 |
| A | JP 2014-68721 A (NICHIBAN CO., LTD.) 21 April 2014, paragraphs [0012]-[0013] & WO 2014/051040 A1 & CN 104780874 A | 1-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 27 May 2019 (27.05.2019) | 04 June 2019 (04.06.2019) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2019/007529

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2001-524357 A (MINNESOTA MINING AND MANUFACTURING COMPANY) 04 December 2001, paragraph [0097], table 3 & US 6171985 B1 & US 6368687 B1, column 22, lines 12-17, table C & WO 1999/027975 A1 & EP 1035876 A | 1-6 |
| A | JP 2000-189453 A (NICHIBAN CO., LTD.) 11 July 2000, paragraph [0021] & US 6297421 B1, column 7, lines 10-21 & EP 1060719 A1 | 1-6 |
| A | JP 2013-248182 A (NICHIBAN CO., LTD.) 12 December 2013, table 2 (Family: none) | 1-6 |
| A | JP 2011-208069 A (BANDO CHEMICAL INDUSTRIES, LTD.) 20 October 2011, paragraph [0020] (Family: none) | 1-6 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014059 A
- JP 9000560 A
- JP 10033585 A
- JP 11009623 A